# EUROPEAN PATENT APPLICATION

(11) **EP 3 906 950 A1**
(43) Date of publication of application: **10.11.2021**
(21) Application number: 20173686.5
(22) Date of filing: 08.05.2020
(51) Int. Cl.: A61L 29/06, A61L 29/08, A61L 29/14, A61F 2/958

(54) **MEDICAL DEVICE DELIVERY SYSTEM WITH IMPROVED MEDICAL DEVICE RETENTION**

(71) Applicant: Bentley InnoMed GmbH, 72379 Hechingen (DE)
(72) Inventor: RIETH, Thomas, 72145 Hirrlingen (DE); KLAUS, Christoph, 72070 Tübingen (DE); STUKOWSKI, Falk, 71277 Rutesheim (DE); BEINRAUCH, Pietro, 96361 Steinbach am Wald (DE)
(74) Representative: Hoefer & Partner Patentanwälte mbB

(57) **Abstract**

The invention relates to a medical device delivery system comprising a catheter shaft having a proximal end and a distal end portion; an expandable member provided at the distal end portion of the shaft, the expandable member having a delivery configuration and a deployed configuration; the expandable member being folded around the catheter shaft in its delivery configuration; characterized in that a coating is disposed on at least a portion of the outer surface area of the expandable member in its delivery configuration such that the coating does not cover portions of the expandable member located inside the folds of the expandable member in its delivery configuration; and an expandable medical device is mounted on the expandable member in the delivery configuration with the coating being disposed between the expandable member and the expandable medical device.

## Description

### BACKGROUND OF THE INVENTION

### Field of the Invention

The present invention generally relates to medical device delivery systems. Particularly, the present invention is directed to a balloon catheter having a coating for improved stent retention, delivery and deployment.

### Description of Related Art

Cardiovascular disease is prevalent in the world. One manifestation of cardiovascular disease is atherosclerosis, which is the buildup of plaque (or fatty deposits) on the walls of blood vessels, such as coronary arteries. Typically, atherosclerosis is treated by percutaneous transluminal coronary angioplasty (PTCA). This procedure generally entails introducing a balloon catheter assembly into the cardiovascular system of a patient via the brachial or femoral artery, and advancing the catheter assembly through the coronary vasculature until the balloon is positioned across an occlusive lesion. Once in position across the lesion, the balloon is inflated to a predetermined size to radially compress against the atherosclerotic plaque of the lesion to remodel the vessel wall. Subsequently, the balloon is deflated to allow the catheter assembly to be withdrawn from the vasculature.

While PCTA is widely used, it suffers from two unique problems. First, the blood vessel may suffer acute occlusion immediately after or within the initial hours after the dilation procedure. Second, restenosis, i.e. re-narrowing of an artery or other blood vessel after an initially successful angioplasty sometimes results.

To reduce the restenosis rate of angioplasty alone and to strengthen the dilated area, physicians typically implant a tubular endoprosthesis, generally called a stent or stent graft, inside the vasculature at the site of the lesion or blocked segment. Stents or stent grafts may also be used to repair vessels having an intimal flap or dissection or to generally strengthen a weakened section of a vessel or to maintain its patency. A typical stent or stent graft delivery system for balloon expandable stents or stent grafts is characterized by a catheter equipped with a dilation balloon and a stent mounted on the balloon, otherwise known as a stent delivery system. In such a system, the stent is slipped over a folded catheter balloon and crimped in place. Additionally, the stent or implantable medical device may be loaded with one or more beneficial agents, such as anti-proliferative agents, for delivery to the target lesion. The stent delivery device enters the vasculature of a patient and travels through a tortuous path to the site of the lesion. The physician positions the stent across the lesion and deploys the stent so that the stent forces the plaque against the inside wall of the blood vessel (or lumen) and maintains its expanded configuration so that the patency of the blood vessel is maintained.

A concern with stent or stent graft deployment, however, is possible slippage and early unintentional release of the stent or stent graft. The stent may release from the balloon during delivery or deployment, such as in small or heavily occluded arteries where contact with either the arterial wall or the lesion to be treated may occur. Additionally, passage of the exposed stent through a valve may cause the stent to be dislodged from the balloon. If the stent is dislodged from or moved relative to the balloon, the system may not be able to correctly implant the stent into body lumen. The steps necessary to remove such a stent can be complicated, and may even require invasive surgery.

Different methods have been attempted to maintain the position of the stent on the expandable member. One such method involves surrounding the catheter and stent assembly with a protective sheath, which is retracted prior to inflation of the expandable member. The use of the sheath, however, increases the profile of the catheter assembly which must traverse narrow vessels. Dissolvable bands or members also have been applied to the stent surface in an effort to hold the stent in place. The bands, however, also add significantly to the outer diameter of the stent assembly and leave exposed and irregular contours of the stent assembly. Other methods to increase stent retention include providing protrusions on the balloon or on the catheter near the balloon, the protrusions having shoulders above and/or below the stent location which bears against the stent when it is subjected to an axial force. Slight inflation of the balloon to fill cells or gaps within the stent also has been employed. Other methods include the provision of elastomeric sleeves on the catheter balloon where the stent is embedded in an outer surface thereof. However, these procedures may be difficult and time consuming, and lead to weakening of the balloon wall, an increase in the pressure required to inflate the balloon, and/or additional manufacturing steps.

Also, catheter balloons are provided with a film-forming polymer coating. EP2676639A1 for example discloses a stent delivery system that can limit / prevent drop-off or shifting of a stent from the balloon and discloses a layer for preventing stent drop-off containing a compound with multiple thiol groups which is formed on at least a portion of the balloon surface. EP0778012A1 discloses a stent delivery system including a dilatation balloon. The system also includes structures integral with the balloon and possibly the catheter for resisting movement of the stent relative to the balloon throughout all phases of a stent delivery. WO2004/020012 discloses a coating composition for use in increasing the static friction of a surface of a delivery system comprising a medical device having a surface in contact with the surface of a delivery component, the static friction of the surface being increased in an amount sufficient to substantially maintain the position of the medical device on the delivery component against forces asserted on the delivery system as it navigates through a vessel of the body. A composition includes a polyether monomer, such as an alkoxy poly (alkylene glycol), a carboxylic acid-containing monomer, such as (meth)acrylic acid, optionally a photoderivatized monomer, and a hydrophilic monomer such as (meth)acrylamide. WO2006/086131 discloses a stent delivery balloon catheter having improved stent retention. A stent mounted on the balloon catheter is embedded in an outer surface of an elastomeric sleeve on the catheter balloon such that the stent forms an imprint in the outer surface of the sleeve. WO2001/76525 is directed to a stent delivery system having a coating selectively applied thereto. The coating may coat all portions of the expandable device except those areas under the stent. The coating may selectively coat areas under the stent in order to aid in stent deliverability and/or deployment or the coating may partially cover the stent in order to aid stent deliverability and stent fixation on the catheter. The coating may be a lubricious material, such as a hydrophilic material. The coating may be applied through various techniques, including the use of templates such as elastic bands that shield selected areas of the catheter.

However, increasing stent retention so far also goes along with an increasing risk of failure in the deployment of the stent as well as failure when retracting the delivery device. Also, most means known in the art to increase the retention of a medical device on its delivery device require expensive technologies or are bulky thus increasing the dimensions of the device in its delivery configuration significantly or, in case a stent graft is mounted on a balloon, massively increase the risk of damaging the membrane of a stent graft during the crimping process.

Accordingly, there is a continued need for an improved method and system for improved stent retention without inhibiting balloon or catheter function and with decreased friction between the balloon and medical device upon balloon deflation and during balloon retraction.

### SUMMARY OF THE INVENTION

To achieve these and other advantages and in accordance with the purpose of the invention, there is provided a medical device delivery system according to claim 1. The medical device delivery system comprises a catheter shaft having a proximal end and a distal end portion and an expandable member being provided at the distal end portion of the shaft. The expandable member has a delivery configuration and a deployed configuration, the expandable member is folded onto the catheter shaft in its delivery configuration and a coating is disposed over at least a portion of the outer surface area of the expandable member in its delivery configuration such that the coating does not cover portions of the expandable member located inside the folds of the expandable member in its delivery configuration. Further, an expandable medical device is mounted on the expandable member in the delivery configuration with the coating disposed between the expandable member and the expandable medical device.

In a preferred embodiment the coating is disposed on the outer surface of the balloon portion constituting the working portion of the balloon.

The coating material preferably comprises a thermoplastic polyurethane elastomer, and more preferably a polycarbonate-based thermoplastic polyurethane elastomer. Preferably, the coating is a spray coating.

In one embodiment of the present invention, the medical device is a stent or a stent graft. Preferably, the expandable member is made of a polymer or copolymer comprising a polyamide. Preferably, the expandable member is a balloon that may be made from a polymer or copolymer comprising a polyamide.

In one embodiment of the present invention the medical device comprises a metallic stent. Preferably, an inner surface of the metallic stent is in direct contact with the coating of the expandable member in its collapsed delivery configuration. Alternatively, the medical device comprises a stent made from a degradable or non-degradable polymer. In another embodiment of the of the present invention, the medical device is a stent graft, the stent graft preferably being a metallic or non-metallic stent being covered with a graft material. In one embodiment the cover of the stent graft may be a membrane made from PTFE, expanded PTFE (ePTFE) or electrospun PTFE like for example Bioweb™ from Zeus Industrial Products, Inc.. In a further embodiment, the membrane may be made from Polyurethane, PET, PE or any combination thereof. The membrane or cover or graft material of the stent graft may cover the whole stent or only a portion thereof. Further the graft material may cover the stent on its outer side or its inner side, also called luminal side, or may cover the outside and the inside of the stent as a whole or only a portion thereof. There are many ways well known in the art to fix the graft material to the stent among them is sewing or gluing the membrane to the stent, spinning the graft material directly on the stent, or clamping the graft material between stent struts embodied to function as clamps.

A method for securing a stent onto a stent delivery system is also provided. In one embodiment, the method comprising the steps of providing an intraluminal catheter device including a catheter shaft having a proximal end portion and a distal end portion with an expandable member disposed at the distal end portion of the shaft; the expandable member having a delivery configuration and a deployed configuration; folding the expandable member onto the catheter shaft to transfer the expandable member in its delivery configuration; disposing a coating on at least a portion of the outer surface area of the expandable member in its delivery configuration such that the coating does not cover portions of the expandable member located inside the folds of the expandable member in its delivery configuration; providing an expandable medical device and mounting the expandable medical device on the expandable member in the delivery configuration with the coating being disposed between the expandable member and the expandable medical device.

In a preferred embodiment, the coating is disposed on at least a portion of the folded medical device by spraying a coating solution onto the folded balloon. In one embodiment, the coating solution comprises a thermoplastic polyurethane elastomer and a solvent and is sprayed onto to at least a portion of the folded balloon. In a preferred embodiment, the coating solution comprises between 10 and 200 g/l, preferably between 50 and 150 g/l, preferably between 75 and 125 g/l, and more preferably between 100 and 125 g/l, preferably 125 mg/l of a polycarbonate-based thermoplastic polyurethane in trichloromethane or hexafluoroisopropanol.

In accordance with another aspect of the invention the folded medical device is rotated along its longitudinal axis during the process of spraying the coating onto at least a portion of the folded balloon.

The accompanying drawings, which are incorporated in and constitute part of this specification, are included to illustrate and provide a further understanding of the product and method of the invention. Together with the description, the drawings serve to explain the principles of the invention.

### BRIEF DESCRIPTION OF THE DRAWINGS

- FIG. 1: is a schematic view of a stent delivery catheter system in accordance with one embodiment of the invention;
- FIG. 2: is a cross-sectional view taken along lines A-A in FIG. 1;
- FIG. 3: is a cross-sectional view taken along lines B-B in FIG. 1;
- FIG. 4: is a cross-sectional view taken according to Fig. 3 of an alternative embodiment.
- FIG. 5: is a cross-sectional view of the stent delivery catheter system of FIG. 1 in a deployed condition with the stent expanded;
- FIG. 6: is a schematic drawing of a spray coating apparatus suitable to coat the folded expandable member.

### DETAILED DESCRIPTION OF THE PREFERRED EMBODIMENT

Reference will now be made in detail to the present preferred embodiments of the invention, examples of which are illustrated in the accompanying drawings. The method and corresponding steps of the invention will be described in conjunction with the detailed description of the intravascular stent delivery catheter device.

In accordance with the present invention an intraluminal medical device delivery system having improved retention of the medical device is provided. Generally, the medical device delivery system includes a catheter shaft having a proximal end and a distal end portion with an expandable member, preferably a medical balloon, provided at the distal end portion of the shaft. The expandable member has a delivery configuration and a deployed configuration. In its delivery configuration the expandable member is folded onto the catheter shaft or wrapped around the catheter shaft and a coating is disposed on at least a portion of the outer surface area of the expandable member such that the coating does not cover portions of the expandable member located inside the folds of the expandable member in its delivery configuration. Further, an expandable medical device, preferably a stent or stent graft, is mounted on the expandable member in the delivery configuration with the coating disposed therebetween.

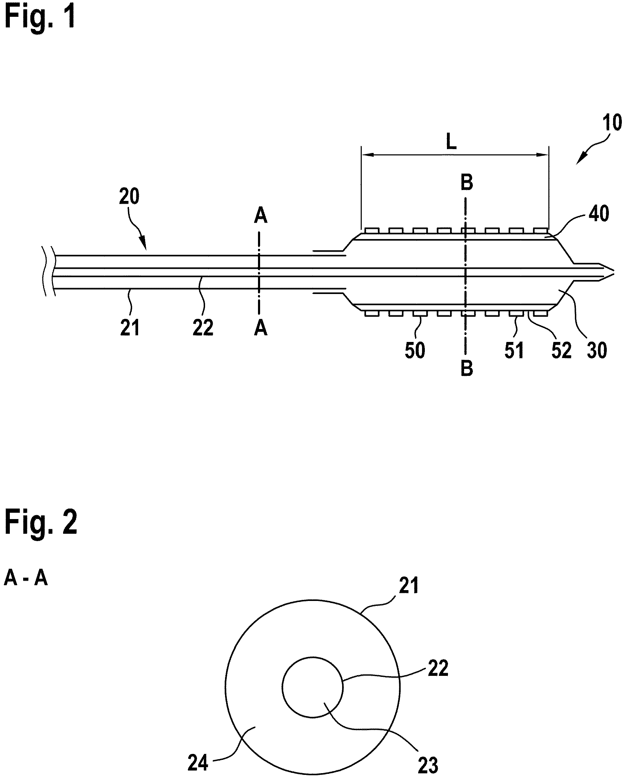

Figs. 1, 2, and 3 schematically illustrate an exemplary embodiment of the medical device delivery system. As shown in FIGS. 1, 2 and 3, the intraluminal medical device 10 generally includes an elongated catheter shaft 20 having a proximal end and a distal end, an expandable member 30 located proximate the distal end of the catheter shaft, and a coating 40 is disposed on the expandable member 30 in its delivery configuration. An expandable stent 50 is mounted on the expandable member 30 with the coating 40 therebetween.

A variety of catheter assemblies, particularly dilatation balloon catheters, are known and suitable for the stent delivery system of the invention. The elongated catheter is sized and configured for delivery through a tortuous anatomy. For purpose of illustration only, the catheter shaft 20 embodied herein comprises an outer tubular member 21 and an inner tubular member 22. As shown in FIG. 2, the inner tubular member 22 defines the guidewire lumen 23 for a guidewire (not shown). Although FIGS. 1 and 2 illustrate the guidewire lumen 23 with an over-the-wire (OTW) configuration, the guidewire lumen 23 can be configured for rapid-exchange (RX) delivery, as is well known in the art. Alternatively, the catheter body can include a fixed guidewire to permit the catheter to be delivered to a vessel location without the use of a separate guidewire if desired.

Between the outer tubular member 21 and the inner tubular member 22 an inflation lumen 24 extending between the proximal end portion and the distal end portion of the catheter shaft 20 is defined. Specifically, as illustrated in FIG. 2, the coaxial relationship between the inner tubular member 22 and the outer tubular member 21 defines an annular inflation lumen 24 therebetween. The expandable member 30 is provided in fluid communication with the inflation lumen 24. The inflation lumen 24 can supply fluid under pressure to expand the expandable member 30 to a deployed condition, and if desired establish negative pressure to deflate the expandable member 30. The expandable member 30 can thus be inflated and deflated using the inflation lumen 24. Suitable materials and techniques are well known for construction of the catheter shaft.

The expandable member can be provided with a variety of configurations and constructions suitable for deployment of an expandable medical device, preferably a stent or a stent graft. Generally, and for purpose of illustration and not limitation, reference is made to an expandable member in the form of a balloon as is well known in the art. As embodied herein, the expandable member generally inflates to a cylindrical configuration with a central working length. The "working length" or "working portion" of the expandable member, or a balloon as embodied herein, is defined as the portion or length upon which the expandable stent is mounted to as described further below. With reference to the balloon embodied herein, the expandable member can be fabricated from one or more polymers (e.g., mixture, blends or layers of polymers). For example, the polymers can include one or more thermoplastics and/or thermoset polymers. Examples of thermoplastics include polyolefins; polyesters (e.g., polyethylene terephthalate (PET), polybutylene terephthalate (PBT), polyethylene naphthalate (PEN), polytrimethylene terephthalate (PTT)); polyethers; polyurethanes; polyvinyls; polyacrylics; fluoropolymers; copolymers and block copolymers thereof, such as block copolymers of polyether and polyamide (e.g., PEBAX); but preferably polyamides (e.g., nylon, such as nylon 12, nylon 11, nylon 6/12, nylon 6, nylon 66); and mixtures thereof. Other examples of polymers that can be used to fabricate the expandable member include polyethylenes, polyethylene ionomers, polyethylene copolymers, polyetheretherketone (PEEK), thermoplastic polyester elastomers (e.g., Hytrel®), and combinations thereof. The expandable member can include multiple layers provided, for example, by coextrusion.

The expandable member can be made using any suitable technique, such as blow molding, film molding, injection molding, and/or extrusion. For example, a polymer tube can be extruded, and can thereafter be stretched and blown to form a balloon.

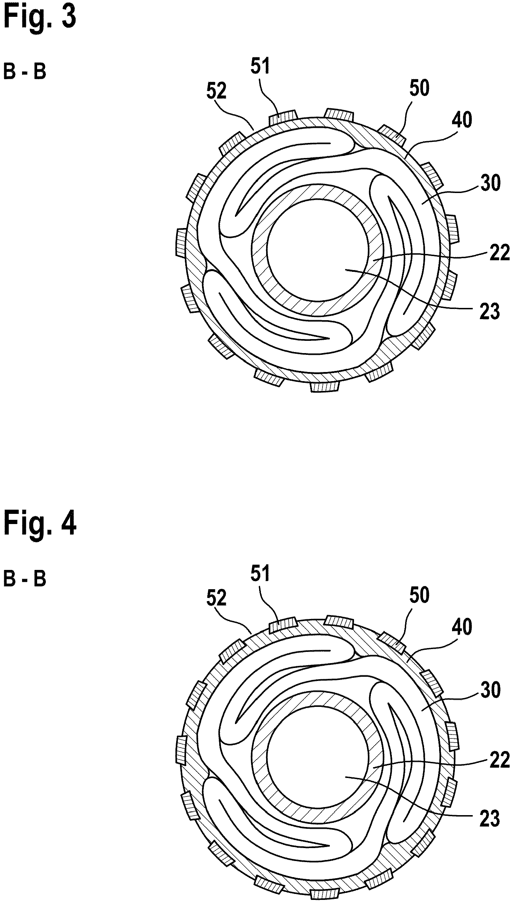

The expandable member has a delivery condition with a reduced profile, and a deployed condition with an expanded profile. As illustrated in FIG. 3, the delivery condition of the expandable member can be accomplished by folding the balloon to a reduced profile. Typically, the expandable member 30 is collapsed down and then folded around the inner tubular member 22. The expandable member 30 can be folded using various techniques well known to those skilled in the art. In one embodiment the expandable member 30 is collapsed down to form folds and the folds are then folded or wrapped around the inner tubular member 22. The folding process can result in an expandable member with several folds, including but not limited to, two, three, four, five or six folds. It has to be noted that the folds of the expandable member in its delivery configuration are not illustrated in FIG. 1.

As noted above, a coating is disposed on at least a portion of the outer surface of expandable member in its collapsed or also called folded or delivery configuration. FIG. 1 shows a coating 40 over the entire length of the working length "L" of the expandable member 30. In alternative embodiments, the coating may also extend over the entire length of the expandable member, and/or over a portion of the catheter shaft. Alternatively, the coating 40 can be provided over only a portion of the working length of the expandable member in its delivery configuration. In a preferred embodiment the coating is disposed on the outer surface of the balloon portion constituting the working portion or working length of the balloon. It is important that the coating only covers those portions of the expandable member that are on the outer surface of the expandable member in its delivery configuration or in its folded configuration. The coating must not be applied to the surface of the expandable member located inside any folds or crinkles of the expandable member when its in its delivery configuration, i.e. there is no coating inside the folds.

In a preferred embodiment of the present invention, the coating is a spray coating, i.e. the coating is applied to the expandable member in its delivery configuration by spraying. Generally, also other coating techniques well known in the art, like e.g. swabbing or painting are also possible to be used, however, spaying is the best method to ensure a uniform coating which is applied only to the outer surface of the expandable member in its delivery configuration, such that the coating does not cover portions of the expandable member located inside the folds of the expandable member in its delivery configuration.

The coating material preferably comprises a thermoplastic polyurethane elastomer, and more preferably a polycarbonate-based thermoplastic polyurethane elastomer. Even more preferably, the coating material consists essentially a thermoplastic polyurethane elastomer, and more preferably a polycarbonate-based thermoplastic polyurethane elastomer.

Examples of suitable thermoplastic polyurethane elastomers are Carbothane PC3575A, Carbothane PC3585A or Carbothane PC3595A (Lubrizol Advanced Materials, Inc.) or mixtures thereof.

In a preferred embodiment of the present invention, the coating has a thickness of 1 to 100 µm, preferably 1 to 50 µm.

The coating is configured to enhance retention of a medical device suitable for implant as described in further detail below. For example, the implantable device can be a stent, a stent graft, a filter, an occlusive device and the like. Furthermore, the stent or medical stent device is not intended to be limited to cardiovascular applications. For example and not limitation, other applications within the scope of the subject matter disclosed herein include spinal or other orthopedic implants, neurovascular or gastrointestinal implants and the like.

In the embodiment depicted in FIGS. 1 and 3, the expandable medical device 50 may be a stent or a stent graft (membrane not shown). The expandable member is a balloon, preferably made of a polymer or copolymer comprising a polyamide. Particularly, FIGS. 1 and 3 show a representative balloon expandable medical device 50 generally comprising a plurality of strut elements 51 defining interstices 52 therebetween. With reference to the expandable medical device embodied herein, the strut elements form a generally tubular body defined by a series of interconnected rings, although a wide array of various stent or other medical device configurations are known and suitable for the subject matter disclosed herein.

The medical device 50 or stent embodied herein is shown in a non- expanded configuration extending along the central, working length of the expandable member 30 and mounted on the coating 40 as illustrated in FIG. 1. In operation, the catheter embodied herein is advanced to a target lesion or area of treatment in the vasculature in a conventional manner with the expandable member in a non-inflated or delivery configuration. After properly positioned within the vasculature, the expandable member 30 is then inflated by directing inflation fluid through an inflation lumen 24 and into the interior of the expandable member 30. In this manner, the expandable member 30 and ultimately the stent 50 are expanded. When expanding the expandable member 30, the folds of the expandable member will unfold to transform the expandable member from its delivery configuration into an expanded configuration. Simulataneously, the expandable member 30 expands the expandable medical device, i.e. the stent 50 (or stent graft) as depicted in FIG. 3. The expandable member 30 is then deflated with the medical device 50 implanted in the vasculature at the target site of treatment.

The stent or other medical device for delivery can be made of any suitable material, such as metal, metal alloy, or polymeric material. Exemplary materials include stainless steel, nitinol, cobalt chromium alloy, ceramics and composites, bioabsorbable polymers, biostable polymers and thermotropic liquid crystal polymers. The stent or medical device to be delivered can be fabricated by utilizing any number of methods known in the art. For example, a stent can be fabricated from a hollow or formed tube that is machined using lasers, electric discharge milling, chemical etching or other known techniques. Alternatively, the stent can be fabricated from a sheet that is rolled into a tubular member, or formed of a toroidal rings, wire or filament construction as known in the art. In a preferred embodiment of the present invention the medical device comprises a metallic stent. Though the stent may also be made from any degradable or nondegradable polymer. In a further preferred embodiment, the medical device is a stent graft, the stent graft preferably being a metallic stent being covered with a graft material as depicted above.

In accordance with the invention, the coating is configured to retain the medical device stent on the expandable member. As illustrated in FIG. 3, the stent 50 is mounted on the outer surface of the coating 40. Although the stent struts are shown with rectangular cross-sections, it is understood that alternative cross-sections are also possible, such as square or circular. In the embodiment illustrated in FIG. 3, the coating is configured such that it is essentially not compressed or not compressed by the stent during the mounting or crimping process. This is the case when the coating is thin like for example but not limitation between 1 µm and 25 µm in thickness. Depending on the crimping process, the balloon material may protrude partially into at least some of the interstices of the stent 50 between the stent struts (not shown) or the stent will sit on the balloon material without any material protrusion. Due to the nonslip surface of the thermoplastic polyurethane polymer coating 40, a general interference between the stent 50 and the coating 40 occurs. This interference provides a mechanical retention force on the stent when removal loads are applied. As a result of the opposing forces provided by the coating, the stent is less likely to dislodge when the removal loads are applied, thereby resulting in improved stent retention. Removal loads may be any forces that try to dislodge the stent off the delivery device like shear forces occurring while navigating through torturous or narrow vessels.

In an alternative embodiment, as depicted in FIG. 4., the coating is designed and configured such that it is compressed by the stent during the mounting and/or crimping process. The coating that is not compressed by the stent will fill at least one or more interstices formed between the adjacent stent struts. This results in an additional interference between the stent 50 and the coating 40 disposed on the outer surface of the expandable member in its delivery configuration and will thus improve stent retention to an even greater extent than in the embodiment depicted in FIG. 3.

In accordance with one embodiment of the invention, as illustrated in FIG. 4, portions of the coating protrude into at least one or more interstices formed between the adjacent stent struts. Such protrusions are in contact with the side surfaces of the stent struts, which define the interstices. In one embodiment of the invention, as illustrated in FIG. 4, the side surfaces of the stent 40 are at least partially encapsulated and the portions of the coating protrude into every interstice formed between the adjacent stent struts. However, in an alternative embodiment, the coating can be configured such that the coating fully protrudes into each interstice and/or does not protrude into every interstice formed between the adjacent stent strut (not illustrated). The characteristics of the coating depend on several characteristics, including but not limited to, material used and thickness of the coating. The degree of protrusion levels can be customized to achieve desired retention characteristics. For example, a large protrusion level can be used when the stent delivery device is intended for a more tortuous path and stent dislodgement is more likely.

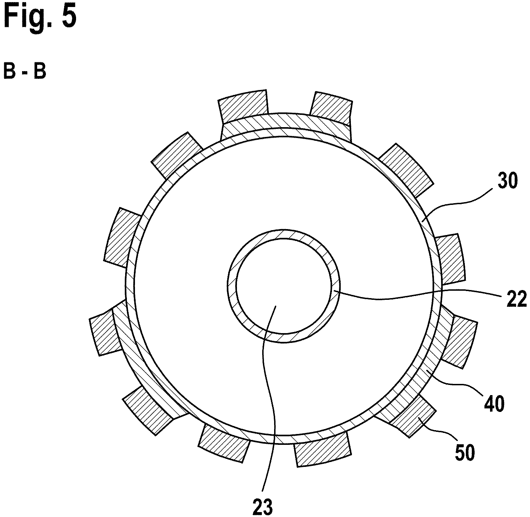

The stent delivery balloon catheter of the present invention is delivered to the desired treatment site. Once the catheter is in place across the site, the expandable member is inflated in a conventional manner. As illustrated in FIG. 5, the expandable member 30 is inflated to its expanded configuration so that the stent 50 is expanded against the lumen wall (not shown). It has to be noted that the coating 40 does not prevent or inhibit the expandable member 30 from inflating and does not constrain the expandable member 30 from inflating. As the stent radially expands to the expanded configuration, as illustrated in FIG. 5, there is a degree of stent deformity as it radially expands. With the expandable member 30 expanding from its delivery configuration to the deployed configuration, the folds of the expandable member unfold. As the coating has been applied to the expandable member in its delivery configuration, the coating covers only distinct portions of the expandable member in its deployed configuration, i.e. only the portions of the expandable member that faces outward or were positioned at the outer surface of the folded expandable member in its delivery configuration are covered with the coating. This results in a widely decreased adhesion of the stent to the expandable member in its deployed configuration. Following inflation of the expandable member and expansion of the stent within the vessel, the expandable member is deflated so that it pulls away from the stent for removal, while the stent stays in its implanted position at the vessel wall.

It will be appreciated that in addition to the characteristics of the coating there are other factors like the crimping process that may be modified to improve the stent retention.

In a further embodiment of the present application, the balloon may be additionally layered by a layer comprising any beneficial agent, such as e.g. an antiproliferative, an anti-inflammatory, an antiplatelet, anticoagulant, antithrombotic, antibiotic, or antioxidant. In order to avoid interference of such a layer and the coating, it is preferred that the layer including the beneficial agent is applied to the surface of the expandable member before folding and crimping to ensure that this layer comprising the beneficial agent is located at least on those portions of the expandable device that are located inside the folds once the expandable member is in its delivery configuration, that is on those portions that are not covered by the coating. This has the advantage that no interference of the layer and the coating may occur in the regions inside the folds, thus preventing any possible destruction or interference of the layer comprising the beneficial agent or the beneficial agent itself in these regions. Also, being placed inside the folds during the delivery process, the beneficial agent is protected from being eluted off the surface of the expandable member during delivery of the catheter to the treatment site.

In accordance with the invention, there is also provided a method for securing a stent onto a stent delivery system. The method comprises the steps of providing an intraluminal catheter device including a catheter shaft having a proximal end portion and a distal end portion with an expandable member disposed at the distal end portion of the shaft; the expandable member having a delivery configuration and a deployed configuration; folding the expandable member onto the catheter shaft to put the expandable member in its delivery configuration; disposing a coating on at least a portion of the outer surface area of the expandable member in its delivery configuration such that the coating does not cover portions of the expandable member located inside the folds of the expandable member in its delivery configuration; providing an expandable medical device and mounting the expandable medical device on the expandable member in the delivery configuration with the coating disposed therebetween.

In a preferred embodiment, the coating is disposed on at least a portion of the folded medical device by spraying a coating solution onto the folded balloon. The coating solution comprises a thermoplastic polyurethane elastomer and a solvent. The solvent can be any solvent suitable to dissolve the thermoplastic polyurethane elastomer. In particular, the solvent can be selected from trichlormethane, dimethylacetamide, methylene chloride, trichloroethane, tetrahydrofurane, and hexafluoroisopropanol, whereas trichloromethane or hexafuoroisopropanol are preferred. In one embodiment, the coating solution contains between 50 and 250 g/L, preferably between 100 and 200 g/L, most preferably 125 g/L thermoplastic polyurethane elastomer. In an even more preferred embodiment, the coating solution comprises 125 g/l or less of a polycarbonate-based thermoplastic polyurethane in trichloromethane or hexafluoroisopropanol.

The coating solution is preferably prepared at a temperature of 50°C or below. The application of the coating solution to the expandable member in its delivery configuration is preferably performed at room temperature.

In accordance with another aspect of the invention the coating is applied to the delivery device by spraying the coating onto delivery device, while the delivery device is rotated.

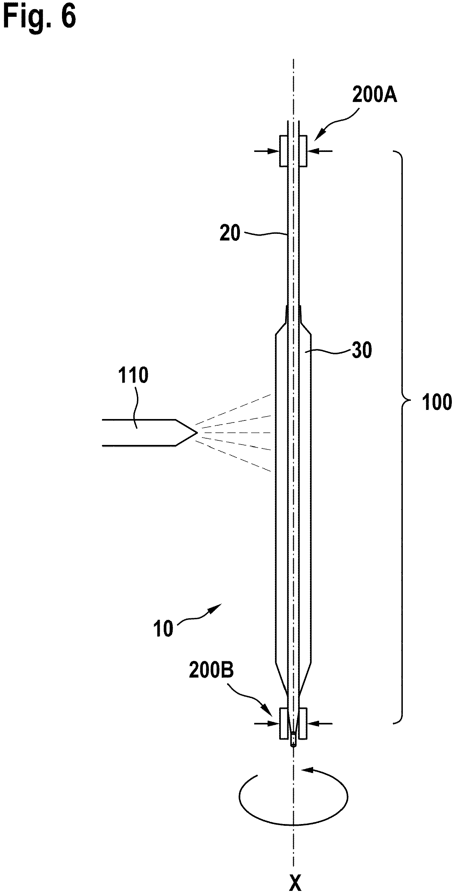

As depicted in FIG. 6, the intraluminal catheter device 10 including a catheter shaft 20 having a proximal end portion and a distal end portion with an expandable member 30 disposed at the distal end portion of the shaft; the expandable member 30 being folded onto the catheter shaft, i.e. the catheter device with the expandable member in its delivery configuration, is affixed to a rotatable apparatus 100 and rotated about its longitudinal axis X. In order to ensure a precise concentric runout, in a preferred embodiment a stylet (not shown) is inserted into a distal portion of the guidewire lumen of the catheter crossing the balloon portion and running along the length of at least the portion of the catheter between the two affixation sites 200A; 200B of the catheter to the rotatable apparatus. While rotating the folded medical device along its longitudinal axis, the coating solution is sprayed onto at least a portion of the expandable member in its delivery configuration. As a spraying or brushing assembly 110 any kind of spray valve suitable to coat the delivery device may be used, though in a preferred embodiment a conventional airbrush system is used. Depending on the working distance, the spraying pressure, the diameter of the spray valve, and the spraying time, the thickness of the coating can be adjusted. By rotation of the delivery device, a uniform coating can be assured. Upon spray coating the coating is dried and subsequently, the medical device is secured on the expandable member as depicted above.

Generally, by spraying multiple very thin coating layers on the folded expandable member, it is assured that no coating solution will enter into the inside portion of the folds of the expandable member. Accordingly, a uniform coating only on the outer surface of the expandable member of the delivery system in its delivery configuration is achieved.

As depicted above, a great advantage of the coating according to the present invention is its easy applicability, its effective stent retention capabilities, and its small dimension.

## Claims

1. A medical device delivery system comprising
a catheter shaft having a proximal end and a distal end portion;
an expandable member provided at the distal end portion of the shaft, the expandable member having a delivery configuration and a deployed configuration;
the expandable member being folded around the catheter shaft in its delivery configuration; **characterized in that**
a coating is disposed on at least a portion of the outer surface area of the expandable member in its delivery configuration such that the coating does not cover portions of the expandable member located inside the folds of the expandable member in its delivery configuration; and
an expandable medical device is mounted on the expandable member in the delivery configuration with the coating being disposed between the expandable member and the expandable medical device.

2. The medical device delivery system of claim 1, wherein the coating is disposed on the outer surface of the balloon portion constituting a working portion of the balloon.

3. The medical device delivery system of claim 1 or 2, wherein the coating is a spray coating.

4. The medical device delivery system of any one of claims 1 to 3, wherein the coating comprises thermoplastic polyurethane elastomer.

5. The medical device delivery system of claim 4, wherein the thermoplastic polyurethane elastomer is a polycarbonate-based thermoplastic polyurethane elastomer.

6. The medical device delivery system of any one of claims 1 to 5, wherein the coating has a thickness of between 1 µm and 100 µm.

7. The medical device delivery system of any one of claims 1 to 6, wherein the medical device is a stent or a stent graft.

8. The medical device delivery system of any one of claims 1 to 7, wherein the medical device comprises a metallic stent.

9. The medical device delivery system of claim 8, wherein an inner surface of the metallic stent is in direct contact with the coating.

10. The medical device delivery system of any one of claims 1 to 9, wherein the expandable member is made of a polymer or copolymer comprising a polyamide.

11. A method for manufacturing a medical device delivery system according to any one of claims 1 to 10, the method comprising the steps of
providing an intraluminal catheter device including a catheter shaft having a proximal end portion and a distal end portion with an expandable member disposed at the distal end portion of the shaft; the expandable member having a delivery configuration and a deployed configuration;
folding the expandable member around the catheter shaft to put the expandable member in its delivery configuration; disposing a coating on at least a portion of the outer surface area of the expandable member in its delivery configuration such that the coating does not cover portions of the expandable member located inside the folds of the expandable member in its delivery configuration;
providing an expandable medical device and mounting the expandable medical device on the expandable member in the delivery configuration with the coating being disposed between the expandable member and the expandable medical device.

12. The method according to claim 11, wherein the coating is disposed on at least a portion of the folded medical device by spraying a coating solution onto the folded balloon.

13. The method according to claim 12, wherein the coating solution comprises a thermoplastic polyurethane elastomer and a solvent and is sprayed onto at least a portion of the folded balloon.

14. The method according to claim 13, wherein the coating solution comprises between 10 and 200 g/l of a polycarbonate-based thermoplastic polyurethane in trichloromethane or hexafluoroisopropanol.

15. The method according to any one of claims 11 to 14, wherein the folded medical device is rotated along its longitudinal axis during the process of spraying the coating onto at least a portion of the folded balloon.
